# EUROPEAN PATENT APPLICATION

(11) **EP 2 088 145 A1**
(43) Date of publication of application: **12.08.2009**
(21) Application number: 07830436.7
(22) Date of filing: 24.10.2007
(51) Int. Cl.: C07D 333/36, C07B 61/00

(54) **METHOD FOR PRODUCING 2-ALKYL-3-AMINOTHIOPHENE DERIVATIVE**

(30) Priority: 10.11.2006 JP 2006304771
(71) Applicant: Mitsui Chemicals, Inc., Tokyo 105-7117 (JP)
(72) Inventor: KAKIMOTO, Takeshi, Omuta-shi Fukuoka 836-8610 (JP); AOKI, Yoji, Omuta-shi Fukuoka 836-8610 (JP); URA, Daisuke, Omuta-shi Fukuoka 836-8610 (JP); ENOMOTO, Miki, Omuta-shi Fukuoka 836-8610 (JP); KAMEKAWA, Hisato, Omuta-shi Fukuoka 836-8610 (JP); KITASHIMA, Toshio, Omuta-shi Fukuoka 836-8610 (JP)
(74) Representative: Wills, Andrew Jonathan
(86) International application number: PCT/JP2007/070702
(87) International publication number: WO 2008/056538

(57) **Abstract**

The present invention provides a method of producing a 2-alkyl-3-aminothiophene derivative represented by formula (2) by reducing at least one of the 2-alkenyl-3-aminothiphene derivatives represented by formulae (1a) to (1d), or a mixture thereof, or a salt thereof, without using a protecting group for an amino group: 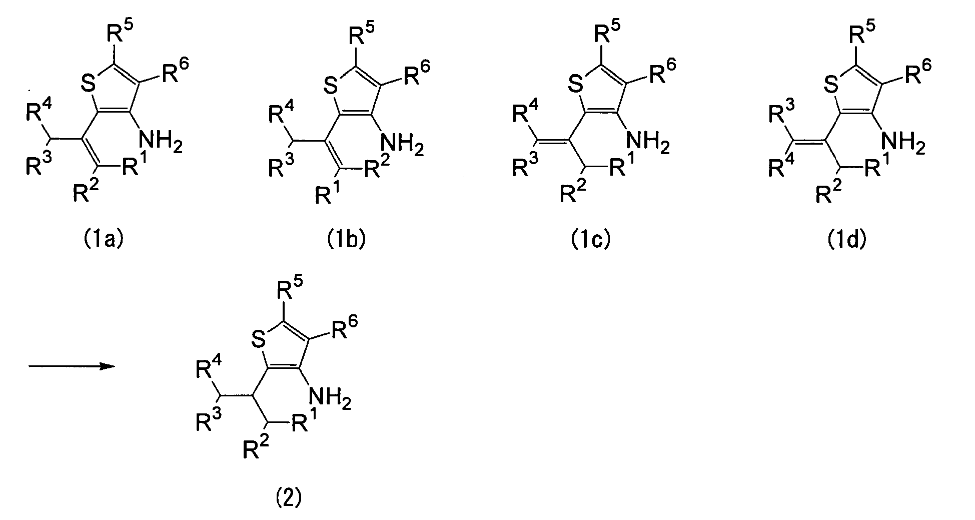 wherein in formulae (1a) to (1d), R¹ to R⁴ each independently represents a hydrogen atom, an alkyl group of 1 to 12 carbon atoms or the like; and R⁵ and R⁶ each independently represents a hydrogen atom, a halogen atom, an alkyl group of 1 to 12 carbon atoms or the like. According to the invention, a 2-alkyl-3-aminothiophene derivative usable as an intermediate of agricultural chemicals can be produced by a cost-efficient industrial process, without the use of a high-cost protective group for an amino group.

## Description

### Technical Field

The present invention relates to a method for producing a 2-alkyl 3-aminothiophene derivative.

### Related Art

Japanese Patent Application Laid-Open (JP-A) No. 9-235282 (European Patent Publication No. 0737682A1) discloses a certain kind of 2-alkyl-3-aminothiophene derivative that exhibits strong effects of controlling various plant diseases, and a method for producing the same. As one of the methods for producing a compound that is a useful intermediate of a 2-alkyl-3-aminothiophene derivative, JP-A No. 2000-327678, for example, discloses a method of reacting a 3-aminothiophene derivative with a ketone of various kinds to synthesize a 2-alkenyl-3-aminothiophene derivative, and reducing the same to synthesize a 2-alkyl-3-aminothiophene derivative. However, in this method described in the above document, as shown in the following Reaction Formula 1, a formyl group, an acyl group, or a carbamate group needs to be used as a protecting group for an amino group, and a process of removing the protecting group also needs to be performed. Therefore, there is room for improvement in ease of operation and economic efficiency.

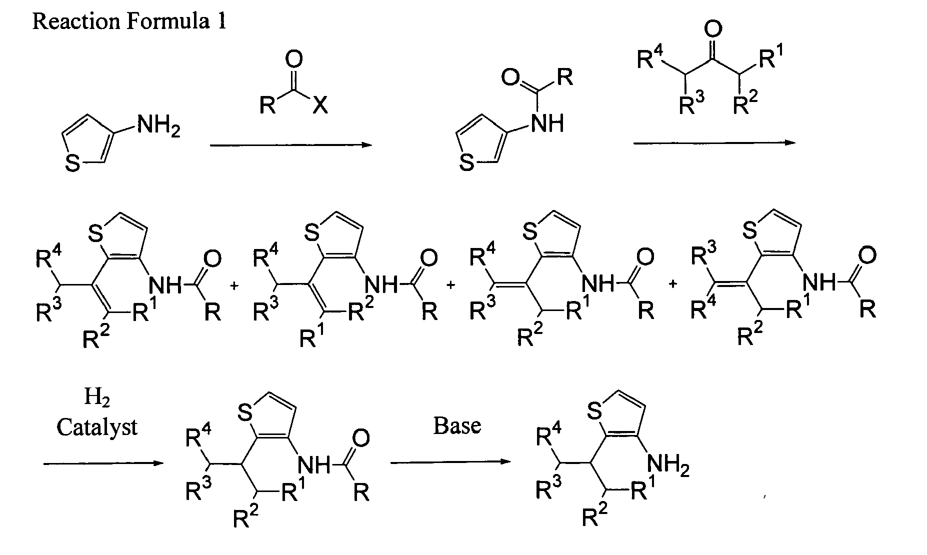

In Reaction Formula 1, R represents a hydrogen atom, an alkyl group or an alkoxy group which may be substituted, an aromatic or non-aromatic hydrocarbon ring which may be substituted, or an aromatic or non-aromatic heterocyclic ring which may be substituted; X represents a halogen atom; R¹, R², R³ and R⁴ each independently represent a hydrogen atom, a straight chain or branched alkyl group having 1 to 12 carbon atoms, where at least one of R¹, R², R³ and R⁴ is a straight chain or branched alkyl group having 1 to 12 carbon atoms, and R¹ and R², R¹ and R³, R¹ and R⁴, R² and R³, R² and R⁴, or R³ and R⁴ may bond to each other to form a cycloalkyl group.

A catalytic reduction method is an industrially advantageous reduction method. However, Japanese Patent Publication No. 8-32702 describes that a thiophene compound generally deactivates all of the hydrogenation catalysts used in the catalytic reduction.
Further, it is known that not only the thiophene compound itself acts as a catalyst poison, but also the catalyst is deactivated by a hydrogen sulfide or a sulfur-containing compound generated by decomposition of the thiophene compound due to a side reaction. When a protecting group is not introduced into an amino group in a 2-alkenyl-3-aminothiophene derivative, the possibility of decomposition may increase due to increased instability, which may lead to reduction in the activity of the catalyst due to poisoning.

Therefore, a method of synthesizing a 2-alkyl-3-aminothiophene derivative by reducing a 2-alkenyl-3-aminothiophene derivative without protecting the amino group thereof in an industrially advantageous manner has not been known hitherto.

### Disclosure of the Invention

### Problems to be Solved by the Invention

The present invention provides a method of producing a 2-alkyl-3-aminothiophene derivative, which is useful as an agricultural chemical intermediate, by reducing a 2-alkenyl-3-aminothiophene derivative without using a protecting group in an industrially cost efficient manner.

### Means for Solving the Problems

In order to solve the above-described problems, the present inventors have extensively researched methods of reducing a 2-alkenyl-3-aminothiophene derivative. As a result, the present inventors have found a method in which a 2-alkenyl-3-aminothiophene derivative can be reduced without using a protecting group for an amino group, which is economically disadvantageous, and have accomplished the present invention as a method of producing a 2-alkyl-3-aminothiophene derivative, which is useful as an agricultural chemical intermediate, in an industrially applicable and cost efficient manner.

More specifically, the present invention relates to the following items 1 to 7.
<1> A method for producing a 2-alkyl-3-aminothiophene derivative, the method comprising reducing a compound represented by any of the following Formulae (1a) to (1d) or a mixture thereof to produce a 2-alkyl-3-aminothiophene derivative represented by the following Formula (2): 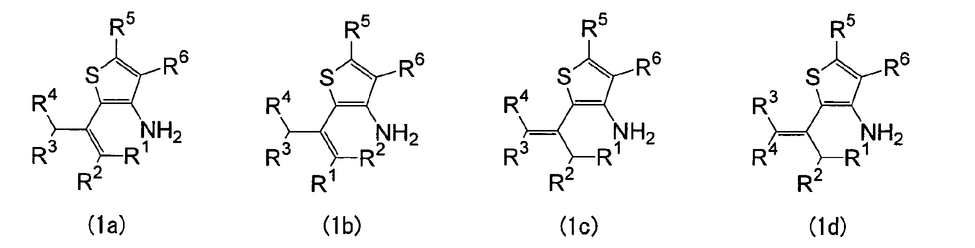 wherein, in Formulae (1a) to (1d), R¹, R², R³ and R⁴ each independently represent a hydrogen atom, an alkyl group having 1 to 12 carbon atoms, or an alkenyl group having 1 to 12 carbon atoms, at least one of R¹, R², R³ and R⁴ being an alkyl group having 1 to 12 carbon atoms or an alkenyl group having 1 to 12 carbon atoms, and R¹ and R², R¹ and R³, R¹ and R⁴, R² and R³, R² and R⁴, or R² and R⁴ being capable of bonding to each other to form a cycloalkyl group; and R⁵ and R⁶ each independently represent a hydrogen atom, a halogen atom, a cyano group, a nitro group, an alkyl group having 1 to 12 carbon atoms, an alkenyl group having 1 to 12 carbon atoms, an alkynyl group having 1 to 12 carbon atoms, a phenyl group, a heterocyclic group, an alkoxy group having 1 to 12 carbon atoms, or an alkylthio group having 1 to 12 carbon atoms, R⁵ and R⁶ being capable of bonding to each other to form a cycloalkyl group; 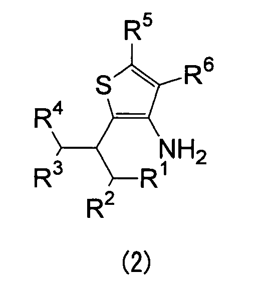 and wherein, in Formula (2), R¹, R², R³, R⁴, R⁵ and R⁶ have the same definitions as in Formulae (1a) to (1d).
<2> The method for producing a 2-alkyl-3-aminothiophene derivative according to < 1>, wherein, in Formulae (1a) to (1d) and Formula (2), R⁵ and R⁶ are a hydrogen atom.
<3> The method for producing a 2-alkyl-3-aminothiophene derivative according to <1> or <2>, wherein, in Formulae (1a) to (1d) and (2), R¹ represents an isopropyl group, and R², R³ and R⁴ are each a hydrogen atom.
<4> The method for producing a 2-alkyl-3-aminothiophene derivative according to any one of <1> to <3>, wherein a salt formed of 2-alkenyl-3-aminothiophene derivatives represented by any of Formulae (1a) to (1d), or a mixture thereof, and an acid, is reduced.
<5> The method for producing a 2-alkyl-3-aminothiophene derivative according to <4>, wherein the acid is an inorganic acid such as hydrogen chloride, hydrogen bromide, sulfuric acid, nitric acid or phosphoric acid, trifluoroacetic acid, cyanoacetic acid, benzoic acid, 4-cyanobenzoic acid, 2-chlorobenzoic acid, 2-nitrobenzoic acid, citric acid, fumaric acid, malonic acid, oxalic acid, maleic acid, phenoxyacetic acid, methanesulfonic acid, p-toluenesulfonic acid, benzenesulfonic acid or p-toluene sulfinic acid.
<6> The method for producing a 2-alkyl-3-aminothiophene derivative according to any one of <1> to <5>, wherein the reducing comprises catalytic reduction.
<7> The method for producing a 2-alkyl-3-aminothiophene derivative according to < 6>, wherein a catalyst used in the catalytic reduction is at least one selected from the group consisting of palladium, platinum, rhodium, ruthenium, nickel, cobalt, chrome, copper, lead and iron.

### Effects of the Invention

According to the present invention, a 2-alkyl-3-aminothiophene derivative that is useful as an intermediate of agricultural chemicals can be produced at low cost by a commercially applicable method by conducting catalytic reduction of a 2-alkenyl-3-aminothiophene derivative in the presence of a catalyst without using a protecting group for an amino group which is economically disadvantageous.

### Best Modes for Carrying Out the Invention

Hereinafter, the present invention will be described in detail.
The reduction reaction in the present invention is represented by the following Reaction Formula (2) in which any one of 2-alkenyl-3-aminothiophene derivatives represented by Formulae (1a) to (1d) or a mixture thereof is reduced to produce a 2-alkyl-3-aminothiophene derivative represented by Formula (2).

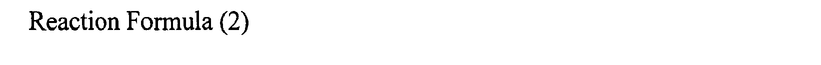

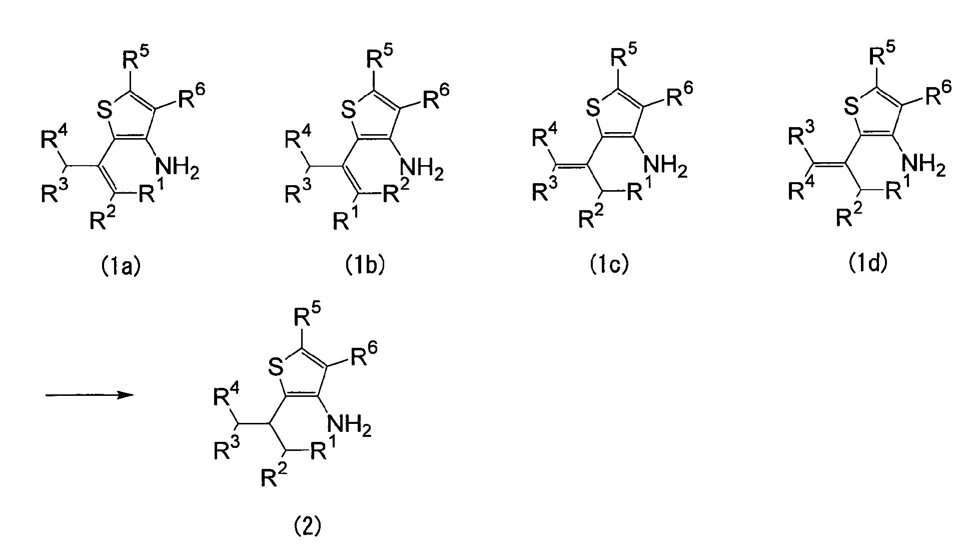

In Reaction Formula (2), R¹, R², R³ and R⁴ each independently represent a hydrogen atom, an alkyl group having 1 to 12 carbon atoms, or an alkenyl group having 1 to 12 carbon atoms, at least one of R¹, R², R³ and R⁴ being an alkyl group having 1 to 12 carbon atoms or an alkenyl group having 1 to 12 carbon atoms, and R¹ and R², R¹ and R³, R¹ and R⁴, R² and R³, R² and R⁴, or R² and R⁴ being capable of bonding to each other to form a cycloalkyl group; R⁵ and R⁶ each independently represent a hydrogen atom, a halogen atom, a cyano group, a nitro group, an alkyl group having 1 to 12 carbon atoms, an alkenyl group having 1 to 12 carbon atoms, an alkynyl group having 1 to 12 carbon atoms, a phenyl group, a heterocyclic group, an alkoxy group having 1 to 12 carbon atoms, or an alkylthio group having 1 to 12 carbon atoms, R⁵ and R⁶ being capable of bonding to each other to form a cycloalkyl group.

In the compounds represented by Formulae (1a) to (1d) and (2), typical examples of the substituent group include, but are not limited thereto, the following groups.

Specifically, examples of an alkyl group having 1 to 12 carbon atoms include a methyl group, an ethyl group, an n-propyl group, an n-butyl group, an n-pentyl group, an n-hexyl group, an isopropyl group, an isobutyl group, a sec-butyl group, a tert-butyl group, and a neopentyl group; examples of a halogen atom include a fluorine atom, a chlorine atom, a bromine atom, and an iodine atom; examples of an alkenyl group having 1 to 12 carbon atoms include a vinyl group, a propenyl group, a butenyl group, a pentenyl group, and a hexenyl group; examples of an alkynyl group having 1 to 12 carbon atoms include an ethynyl group, a propynyl group, a butynyl group, a pentynyl group, and a hexynyl group; examples of an alkoxy group having 1 to 12 carbon atoms include a methoxy group and an ethoxy group; examples of an alkylthio group having 1 to 12 carbon atoms include a methylthio group and an an ethylthio group; examples of a heterocyclic group include furan, thiophene, oxazole, pyrrole, 1H-pyrazole, 3H-pyrazole, imidazole, thiazole, oxazole, isoxazole, isothiazole, tetrahydrofuran, pyrazolidine, pyridine, pyrane, pyrimidine, or pyrazine.
Examples of substituent groups for the alkyl group having 1 to 12 carbon atoms, alkenyl group having 1 to 12 carbon atoms, alkynyl group having 1 to 12 carbon atoms, phenyl group, and heterocyclic group include an alkyl group, such as a methyl group, an ethyl group, an isopropyl group, or an isobutyl group, an alkenyl group, such as a vinyl group or a propenyl group, an alkynyl group, such as an ethynyl group or a propynyl group, an alkyl halide group, such as a trifluoromethyl group, an alkoxy group, such as a methoxy group or an ethoxy group, a halogen-substituted alkoxy group, such as a trifluoromethoxy group or a difluoromethoxy group, an alkylthio group, such as a methylthio group or an ethylthio group, an alkylsulfinyl group, such as a methane sulfinyl group or an ethane sulfinyl group, a halogen-substituted alkyl sulfinyl group, such as a trifluoromethane sulfinyl group or a difluoromethane sulfinyl group, an alkyl sulfonyl group, such as a methane sulfonyl group or an ethane sulfonyl group, a halogen-substituted alkyl sulfonyl group, such as a trifluoromethane sulfonyl group or a difluoromethane sulfonyl group, a phenyl group, a naphthyl group, a heterocyclic group, such as furan, thiophene, oxazole, pyrrole, 1H-pyrazole, 3H-pyrazole, imidazole, thiazole, oxazole, isoxazole, isothiazole, tetrahydrofuran, pyrazolidine, pyridine, pyrane, pyrimidine, or pyrazine, and a halogen atom, such as a fluorine atom, a chlorine atom, a bromine atom, or an iodine atom.
In Formulae (1a) to (1d) and (2), R¹ is preferably an isopropyl group, and R², R³, R⁴, R⁵ and R⁶ are preferably a hydrogen atom.

The 2-alkenyl-3-aminothiophene derivative represented by Formulae (1a) to (1d) used as a raw material in this reaction include up to four kinds of compounds. For example, when all substituents R¹ to R⁴ in Formulae (1a) to (1d) are different from each other, the 2-alkenyl-3-aminothiophene derivative include four kinds of compounds. When R¹ is an isopropyl group and R², R³, R⁴, R⁵ and R⁶ are a hydrogen atom, the corresponding 2-alkenyl-3-aminothiophene derivative includes three kinds of compounds represented by the following Formulae (1a') to (1c').

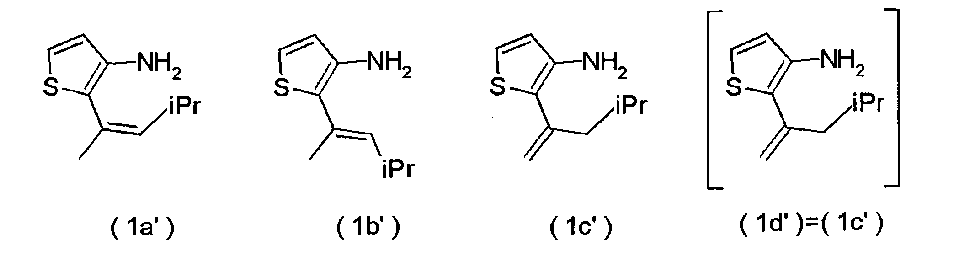

These mixtures can be separated from each other using a techniques such as chromatography, and can be utilized as a raw material for the reaction represented by Reaction Formula (2) as a single compound or as a mixture. It is also possible to use a salt formed from an acid and any one of the 2-alkenyl-3-aminothiophene derivatives represented by Formulae (1a) to (1d) or a mixture thereof in the reaction.

Typical examples of the acid that forms a salt with the 2-alkenyl-3-aminothiophene derivative represented by Formulae (1a) to (1d) or the 2-alkyl-3-aminothiophene derivative represented by Formula (2) include, but are not limited to the following substances, an inorganic acid, such as hydrogen chloride, hydrogen bromide, sulfuric acid, nitric acid, and phosphoric acid, and an organic acid, such as trifluoroacetic acid, cyanoacetic acid, benzoic acid, 4-cyanobenzoic acid, 2-chlorobenzoic acid, 2-nitrobenzoic acid, citric acid, fumaric acid, malonic acid, oxalic acid, maleic acid, phenoxyacetic acid, methanesulfonic acid, p-toluenesulfonic acid, benzenesulfonic acid, and p-toluene sulfinic acid.

The reduction method in the above-described reaction is not particularly limited and a method of reducing a double bond to a single bond may be generally applied (see, for example, Shin Jikken Kagaku Koza (New Experimental Chemistry Course), vol. 15, Sanka to Kangen (Oxidation and Reduction) [II], published by Maruzen (1977)). From an industrial aspect, catalytic reduction is preferable.

The catalyst used in the present invention may be a metal catalyst that is generally used for catalytic reduction, and examples thereof include, but are not limited thereto, palladium, platinum, rhodium, ruthenium, nickel, cobalt, chromium, copper, lead, and iron, and a mixture of these metals may also be used. These metal catalysts may be used in the form of a metal, but are generally supported by a carrier, such as activated carbon, barium sulfate, silica gel, alumina, and celite. Nickel, cobalt, copper, or the like may also be used as a Raney catalyst. The amount of a catalyst used in the reaction is generally from 0.1 to 200% by weight, preferably from 0.5 to 30% by weight, with respect to the compound represented by Formulae (1a) to (1d) or a mixture thereof.

The reaction of the present invention may be carried out in the absence of a solvent, but may also be carried out under dilution with a solvent, as required. Examples of the solvent to be used include, but are not limited to the following substances, alcohols, such as methanol, ethanol, isopropyl alcohol, and octanol, aliphatic hydrocarbon, such as n-hexane and cyclohexane, aromatic groups, such as benzene and toluene, ethers, such as dioxane, tetrahydrofuran, and diethyl ether, esters, such as ethyl acetate and butyl acetate, aliphatic carboxylic acids, such as acetic acid and propionic acid, a ketone solvent, such as methyl isobutyl ketone, and an aprotic polar solvent, such as dimethylformamide and dimethylsulfoxide. A mixture thereof may also be used. The solvent is used at an amount of generally from 0 to 200 ml, preferably 1 to 20 ml, with respect to 1 g of the compound represented by Formulae (1a) to (1d) or a mixture thereof.

The reaction temperature and reaction time for the reaction may be changed in a wide range. In general, the reaction temperature is preferably from -78 to 300°C and more preferably from 20 to 250°C. The reaction time is preferably 0.01 to 100 hours and more preferably 1 to 50 hours.

The reaction pressure for the reaction may be changed in a wide range, and the reaction may be carried out either under normal pressure or under applied pressure. When the reaction is carried out under applied pressure, the reaction pressure is from 0.01 to 30 MPa and preferably from 0.5 to 3 MPa.

The conditions of various kinds with respect to the above-described catalytic reduction reaction (i.e., the type and amount of catalyst, the type and amount of solvent, the reaction temperature, the reaction time, and the reaction pressure) may be appropriately selected from the ordinary range or preferable range thereof and combined with each other.

Examples
The present invention will be described in more detail with reference to the following Examples, but the present invention is not limited thereto.

Example 1
A mixture of three kinds of compounds of 3-amino-2-{(E)-(4-methyl-2-penten-2-yl)}thiophene, 3-amino-2-{(Z)-(4-methyl-2-penten-2-yl)}thiophene, and 3-amino-2-(4-methyl-1-penten-2-yl)thiophene (hereinafter, abbreviated as ATU in the following Examples) (10.09 g) was dissolved in 1-octanol (90.08 g), and a 5% palladium carbon catalyst (water-containing product, water content: 59.20%, 0.51 g) was added thereto. The resulting mixture was sealed in a pressure reactor (capacity: 300 ml). A process of applying a pressure of 0.2 MPa with nitrogen and discharging the same was repeated for 4 times to purge the inside of the reactor with nitrogen, and then a process of applying a pressure of 1.0 MPa with hydrogen and discharging the same was repeated for 4 times to purge the inside of the reactor with hydrogen. The reactor was pressurized with hydrogen to 1.5 MPa and the temperature thereof was increased to 200°C, to allow the content to react over 3 hours. The reactor was cooled at room temperature, and a process of applying a pressure of 0.2 MPa with nitrogen and discharging the same was repeated for 5 times to purge the inside of the reactor with nitrogen. The reactor was opened and the catalyst was removed from the reaction solution by filtration, and then the catalyst was washed with 1-octanol. The filtrate and the 1-octanol used for the washing were analyzed by an HPLC internal standard method. As a result, 3-amino-2-(4-methylpentan-2-yl)thiophene (hereinafter, abbreviated as ATA in the following Examples) was obtained at a yield of 86.02%.

Example 2
ATU (10.00 g) was dissolved in 1-octanol (90.03 g), and a 5% palladium carbon catalyst (water-containing product, water content: 50.18%, 1.02 g) was added thereto. The resulting mixture was sealed in a pressure reactor (capacity: 300 ml). A process of applying a pressure of 0.2 MPa with nitrogen and discharging the same was repeated for 4 times to purge the inside of the reactor with nitrogen, and a process of applying a pressure of. Then, a process of applying a pressure of 1.0 MPa with hydrogen and discharging the same was repeated for 4 times to purge the inside of the reactor with hydrogen. The reactor was pressurized with hydrogen to 1.5 MPa and the temperature thereof was increased to 200°C, to allow the content to react over 3 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with 1-octanol. The filtrate and the 1-octanol used for the washing were analyzed by an HPLC internal standard method. As a result, ATA was obtained at a yield of 85.93%.

Example 3
ATU (150.02 g) was dissolved in 1-octanol (1350.91 g), and a 5% palladium carbon catalyst (water-containing article, water content: 50.18%, 5.98 g) was added thereto. The resulting mixture was sealed in a pressure reactor (capacity: 2000 ml). A process of applying a pressure of 0.2 MPa with nitrogen and discharging the same was repeated for 4 times to purge the inside of the reactor with nitrogen, and a process of applying a pressure of 1.0 MPa with hydrogen and discharging the same was repeated for 4 times to purge the inside of the reactor with hydrogen. The reactor was pressurized with hydrogen to 1.5 MPa and the temperature thereof was increased to 200°C, to allow the content to react over 3 hours. The reactor was cooled to room temperature, and a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with 1-octanol. The filtrate and the 1-octanol used for the washing were purified by distillation under reduced pressure, thereby obtaining ATA (113.76 g) as a colorless oily substance (yield: 75%, bp:129°C, 16 mmHg).

Example 4
ATU (2.58 g) was dissolved in 1-octanol (22.59 g), and a 5% palladium carbon catalyst (water-containing product, water content: 50.18%, 0.57 g) was added thereto. The resulting mixture was sealed in a pressure reactor (capacity: 190 ml). A process of applying a pressure of 0.2 MPa with nitrogen and discharging the same was repeated for 5 times to purge the inside of the reactor with nitrogen. The reactor was pressurized with hydrogen to 1.5 MPa and the temperature thereof was increased to 150°C, to allow the content to react over 3 hours. The reactor was cooled to room temperature, and a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with 1-octanol. The filtrate and the 1-octanol used for the washing were analyzed by an HPLC internal standard method. As a result, ATA was obtained at a yield of 83.53%.

Example 5
ATU (5.00 g) was dissolved in 1-octanol (95.09 g), and a 5% palladium carbon catalyst (water-containing product, water content: 59.20%, 1.24 g) was added thereto. The resulting mixture was sealed in a pressure reactor (capacity: 190 ml). A process of applying a pressure of 0.2 MPa with nitrogen and discharging the same was repeated for 5 times to purge the inside of the reactor with nitrogen. The reactor was pressurized with hydrogen to 1.0 MPa and the temperature thereof was increased to 200°C, to allow the content to react over 3 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with 1-octanol. The filtrate and the 1-octanol used for the washing were analyzed by an HPLC internal standard method. As a result, ATA was obtained at a yield of 92.53%.

Example 6
ATU (0.20 g) was dissolved in ethanol (1.80 g), and a 5% palladium-0.01% lead carbon catalyst (water-containing product, water content: 50.40%, 0.09 g) was added thereto. The resulting mixture was sealed in a pressure reactor (capacity: 8 ml). A process of applying a pressure of 0.2 MPa with nitrogen and discharging the same was repeated for 5 times to purge the inside of the reactor with nitrogen. The reactor was pressurized with hydrogen to 1.0 MPa and the temperature thereof was increased to 100°C, to allow the content to react over 5 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with ethanol. The filtrate and the ethanol used for the washing were analyzed by an HPLC internal standard method. As a result, ATA was obtained at a yield of 37.76%.

Example 7
ATU (0.20 g) was dissolved in ethanol (1.80 g), and a 2.5% palladium-2.5% ruthenium carbon catalyst (water-containing product, water content: 56.28%, 0.08 g) was added thereto. The resulting mixture was sealed in a pressure reactor (capacity: 8 ml). A process of applying a pressure of 0.2 MPa with nitrogen and discharging the same was repeated for 5 times to purge the inside of the reactor with nitrogen. The reactor was pressurized with hydrogen to 1.0 MPa and the temperature thereof was increased to 100°C, to allow the content to react over 5 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with ethanol. The filtrate and the ethanol used for the washing were analyzed by an HPLC internal standard method. As a result, ATA was obtained at a yield of 19.97%.

Example 8
ATU (0.20 g) was dissolved in ethanol (1.80 g), and a 3% platinum carbon catalyst (water-containing product, water content: 63.25%, 0.02 g) was added thereto. The resulting mixture was sealed in a pressure reactor (capacity: 8 ml). A process of applying a pressure of 0.2 MPa with nitrogen and discharging the same was repeated for 5 times to purge the inside of the reactor with nitrogen. The reactor was pressurized with hydrogen to 1.0 MPa and the temperature thereof was increased to 100°C, to allow the content to react over 5 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with ethanol. The filtrate and the ethanol used for the washing were analyzed by an HPLC internal standard method. As a result, ATA was obtained at a yield of 28.98%.

Example 9
ATU (0.20 g) was dissolved in ethanol (1.80 g), and a 5% ruthenium carbon catalyst (water-containing product, water content: 52.79%, 0.08 g) was added thereto. The resulting mixture was sealed in a pressure reactor (capacity: 8 ml). A process of applying a pressure of 0.2 MPa with nitrogen and discharging the same was repeated for 5 times to purge the inside of the reactor with nitrogen. The reactor was pressurized with hydrogen to 1.0 MPa and the temperature thereof was increased to 100°C, to allow the content to react over 5 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with ethanol. The filtrate and the ethanol used for the washing were analyzed by an HPLC internal standard method. As a result, ATA was obtained at a yield of 16.05%.

Example 10
ATU (0.20 g) was dissolved in ethanol (1.80 g), and a 5% platinum + 0.05% tin carbon catalyst (water-containing product, water content: 50.12%, 0.08 g) was added thereto. The resulting mixture was sealed in a pressure reactor (capacity: 8 ml). A process of applying a pressure of 0.2 MPa with nitrogen and discharging the same was repeated for 5 times to purge the inside of the reactor with nitrogen. The reactor was pressurized with hydrogen to 1.0 MPa and the temperature thereof was increased to 100°C, to allow the content to react over 5 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with ethanol. The filtrate and the ethanol used for the washing were analyzed by an HPLC internal standard method. As a result, ATA was obtained at a yield of 36.32%.

Example 11
ATU phosphate (0.31 g) was dissolved in ethanol (2.00 g), and a 5% palladium carbon catalyst (water-containing product, water content: 57.50%, 0.07 g) was added thereto. The resulting mixture was sealed in a pressure reactor (capacity: 8 ml). A process of applying a pressure of 0.2 MPa with nitrogen and discharging the same was repeated for 5 times to purge the inside of the reactor with nitrogen. The reactor was pressurized with hydrogen to 1.0 MPa and the temperature thereof was increased to 100°C, to allow the content to react over 5 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with ethanol. The filtrate and the ethanol used for the washing were analyzed by an HPLC internal standard method. As a result, ATA was obtained at a yield of 19.20%.

Example 12
ATU 1/2 oxalate (0.74 g) was dissolved in methanol (2.00 g), and a 5% palladium carbon catalyst (water-containing product, water content: 57.50%, 0.07 g) was added thereto. The resulting mixture was sealed in a pressure reactor (capacity: 8 ml). A process of applying a pressure of 0.2 MPa with nitrogen and discharging the same was repeated for 5 times to purge the inside of the reactor with nitrogen. The reactor was pressurized with hydrogen to 1.0 MPa and the temperature thereof was increased to 100°C, to allow the content to react over 5 hours. The reactor was cooled to room temperature, and then a process of applying a pressure of 0.2 MPa with nitrogen and discharging the same was repeated for 5 times to purge the inside of the reactor with nitrogen. The reactor was opened and the catalyst was removed from the reaction solution by filtration, and the catalyst was washed with methanol. The filtrate and the methanol used for the washing were analyzed by an HPLC internal standard method. As a result, ATA was obtained at a yield of 16.30%.

## Claims

1. A method for producing a 2-alkyl-3-aminothiophene derivative, the method comprising reducing a compound represented by any of the following Formulae (1a) to (1d) or a mixture thereof to produce a 2-alkyl-3-aminothiophene derivative represented by the following Formula (2): 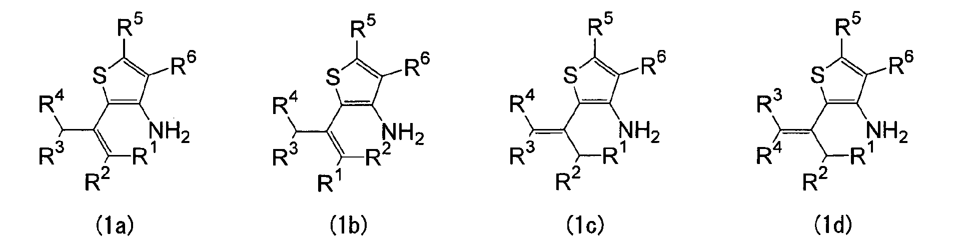 wherein, in Formulae (1a) to (1d), R¹, R², R³ and R⁴ each independently represent a hydrogen atom, an alkyl group having 1 to 12 carbon atoms, or an alkenyl group having 1 to 12 carbon atoms, at least one of R¹, R², R³ and R⁴ being an alkyl group having 1 to 12 carbon atoms or an alkenyl group having 1 to 12 carbon atoms, and R¹ and R², R¹ and R³, R¹ and R⁴, R² and R³, R² and R⁴, or R² and R⁴ being capable of bonding to each other to form a cycloalkyl group; and R⁵ and R⁶ each independently represent a hydrogen atom, a halogen atom, a cyano group, a nitro group, an alkyl group having 1 to 12 carbon atoms, an alkenyl group having 1 to 12 carbon atoms, an alkynyl group having 1 to 12 carbon atoms, a phenyl group, a heterocyclic group, an alkoxy group having 1 to 12 carbon atoms, or an alkylthio group having 1 to 12 carbon atoms, R⁵ and R⁶ being capable of bonding to each other to form a cycloalkyl group; 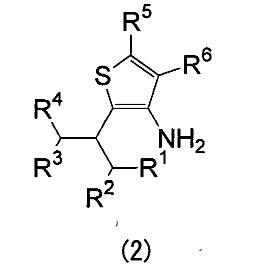 and wherein, in Formula (2), R¹, R², R³, R⁵ and R⁶ have the same definitions as in Formulae (1a) to (1d).

2. The method for producing a 2-alkyl-3-aminothiophene derivative according to claim 1, wherein, in Formulae (1a) to (1d) and Formula (2), R⁵ and R⁶ are a hydrogen atom.

3. The method for producing a 2-alkyl-3-aminothiophene derivative according to claim 1 or 2, wherein, in Formulae (1a) to (1d) and (2), R¹ represents an isopropyl group, and R², R³ and R⁴ are each a hydrogen atom.

4. The method for producing a 2-alkyl-3-aminothiophene derivative according to any one of claims 1 to 3, wherein a salt formed of 2-alkenyl-3-aminothiophene derivatives represented by any of Formulae (1a) to (1d), or a mixture thereof, and an acid, is reduced.

5. The method for producing a 2-alkyl-3-aminothiophene derivative according to claim 4, wherein the acid is an inorganic acid such as hydrogen chloride, hydrogen bromide, sulfuric acid, nitric acid or phosphoric acid, trifluoroacetic acid, cyanoacetic acid, benzoic acid, 4-cyanobenzoic acid, 2-chlorobenzoic acid, 2-nitrobenzoic acid, citric acid, fumaric acid, malonic acid, oxalic acid, maleic acid, phenoxyacetic acid, methanesulfonic acid, p-toluenesulfonic acid, benzenesulfonic acid or p-toluene sulfinic acid.

6. The method for producing a 2-alkyl-3-aminothiophene derivative according to any one of claims 1 to 5, wherein the reducing comprises catalytic reduction.

7. The method for producing a 2-alkyl-3-aminothiophene derivative according to claim 6, wherein a catalyst used in the catalytic reduction is at least one selected from the group consisting of palladium, platinum, rhodium, ruthenium, nickel, cobalt, chrome, copper, lead and iron.
